(19) Europäisches Patentamt
European Patent Office
Office européen des brevets

(11) **EP 4 039 790 A1**

(12) **EUROPEAN PATENT APPLICATION**
published in accordance with Art. 153(4) EPC

(43) Date of publication:
**10.08.2022  Bulletin 2022/32**

(21) Application number: **20872671.1**

(22) Date of filing: **30.09.2020**

(51) International Patent Classification (IPC):
**C12M 1/34** (2006.01)          **G01N 21/17** (2006.01)
**G01N 21/80** (2006.01)          **G01N 33/48** (2006.01)
**G01N 33/483** (2006.01)

(52) Cooperative Patent Classification (CPC):
**C12M 1/34; G01N 21/17; G01N 21/80; G01N 33/48;
G01N 33/483**

(86) International application number:
**PCT/JP2020/037239**

(87) International publication number:
**WO 2021/066041 (08.04.2021 Gazette 2021/14)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority:  **04.10.2019  JP 2019184161**

(71) Applicant: **Kyocera Corporation
Kyoto-shi Kyoto 612-8501 (JP)**

(72) Inventor: **WATANABE, Masaya
Kyoto-shi, Kyoto 612-8501 (JP)**

(74) Representative: **TBK
Bavariaring 4-6
80336 München (DE)**

(54)    **PH MEASUREMENT METHOD AND PH MEASUREMENT DEVICE**

(57)    A method and a device allow measurement of the pH value of a solution. The method includes illuminating a solution containing a pH indicator and a pH changer with light emitted from a light emitting element, receiving light transmitted through the solution with a light receiving element, measuring absorbance with monochromatic light selectively from the received transmitted light, and calculating a pH value corresponding to the measured absorbance based on a predefined absorbance table. The device includes an illuminator that illuminates a solution containing a pH indicator and a pH changer with light emitted from a light emitting element, a light receiver that receives light transmitted through the solution, a measurer that measures absorbance with light with a wavelength of monochromatic light selectively from the received transmitted light, and a calculator that calculates a pH value corresponding to the absorbance measured by the measurer based on a predefined absorbance table.

FIG. 1

## Description

## FIELD

The present disclosure relates to a pH measurement method and a pH measurement device for measuring the pH of a solution.

## BACKGROUND

The pH value of a solution is a major factor in determining the conditions of the solution. For example, the pH value of a cell culture solution is measured to determine whether the solution has deteriorated and to be replaced. The pH value of a solution is measured with a detector of a known pH meter placed in the solution. Also, a substance that changes its color in accordance with the pH value of a solution (pH indicator) is added to a solution in advance. A color change in the solution is then observed visually or using a sensor to obtain the pH value corresponding to the color of the solution. For example, known techniques use a phenol red reagent as a pH indicator to observe a color reaction of the phenol red reagent contained in a cell culture solution, and measure the absorbance of the cell culture solution with light with multiple wavelengths to obtain the pH value corresponding to the measured absorbance (refer to, for example, Patent Literatures 1 to 3).

## CITATION LIST

## PATENT LITERATURE

Patent Literature 1: Japanese Patent No. 5797911
Patent Literature 2: Japanese Unexamined Patent Application Publication No. 2019-106944
Patent Literature 3: Japanese Unexamined Patent Application Publication No. 2019-106945

## BRIEF SUMMARY

## TECHNICAL PROBLEM

However, such known techniques involve lengthy visual observation of the conditions of a solution. Measuring large amounts of solution in many containers can place a large burden on the operator and increase the operation time. Also, a detector of a known pH meter placed in a solution to measure the pH value of the solution may contaminate the solution. A disposable detector of a pH meter to eliminate concerns over contaminated solution is costly. Managing large amounts of solution in many containers with such disposable detectors can be uneconomical. The known techniques described in Patent Literatures 1 to 3 use light with multiple wavelengths with costly equipment including multiple sensors for different wavelengths. These techniques thus involve high facility costs.

The development in bioengineering may allow industrialized cell culture, which expects efficient and inexpensive management of solutions in many containers to culture cells on a larger scale.

Inexpensive pH measurement methods and pH measurement devices that can efficiently measure the pH value of a solution free of concerns over contaminated solutions are thus awaited.

## SOLUTION TO PROBLEM

A pH measurement method according to one or more embodiments of the present disclosure includes illuminating a solution containing a pH indicator and a pH changer with light emitted from a light emitting element, receiving light transmitted through the solution with a light receiving element, measuring absorbance with monochromatic light selectively from the received transmitted light, and calculating a pH value corresponding to the measured absorbance based on a predefined absorbance table.

A pH measurement device according to one or more embodiments of the present disclosure includes an illuminator that illuminates a solution containing a pH indicator and a pH changer with light emitted from a light emitting element, a light receiver that receives light transmitted through the solution, a measurer that measures absorbance with monochromatic light selectively from the received transmitted light, and a calculator that calculates a pH value corresponding to the absorbance measured by the measurer based on a predefined absorbance table.

## ADVANTAGEOUS EFFECTS

The pH measurement method and the pH measurement device according to one or more embodiments of the present disclosure with the above structure allow measurement of absorbance with monochromatic light selectively from the received transmitted light using a single sensor without using multiple color filters and sensors corresponding to light with multiple wavelengths. The pH measurement method and the pH measurement device can be simplified. The pH measurement device with the simplified structure can be provided at low costs. The above structure eliminates placement of a PH meter in a solution, thus eliminating contamination of the solution and allowing inexpensive and easy measurement of the pH value of the solution. The above structure also allows inexpensive and rapid measurement of the pH value of a large amount of solution in many containers.

## BRIEF DESCRIPTION OF DRAWINGS

FIG. 1 is a flowchart of an example pH measurement method according to an embodiment of the present disclosure.

FIG. 2 is a diagram of example quantum efficiency spectra of photodiodes according to the embodiment of the present disclosure.

FIG. 3 is a schematic diagram of the pH measurement method according to the embodiment of the present disclosure.

FIG. 4 is a diagram of example absorption spectra of culture solutions according to the embodiment of the present disclosure.

FIG. 5 is a graph showing the relationship between the pH and the absorbance of a solution according to the embodiment of the present disclosure.

FIG. 6 is a flowchart of an example pH measurement method according to the embodiment of the present disclosure.

FIG. 7 is a block diagram of an example pH measurement device according to the embodiment of the present disclosure.

FIG. 8 is a block diagram of an example pH measurement device according to the embodiment of the present disclosure.

## DETAILED DESCRIPTION

Detection Method

First Embodiment

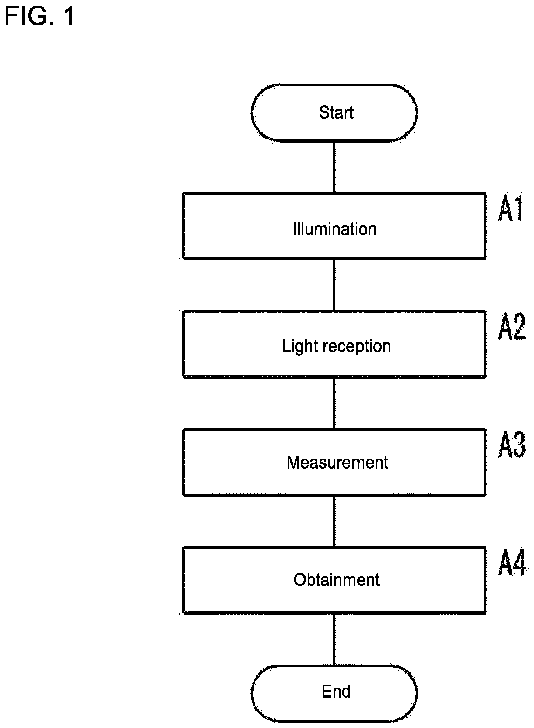

FIG. 1 is a flowchart of a pH measurement method according to a first embodiment. The pH measurement method according to the first embodiment includes illuminating a solution containing a pH indicator and a pH changer with light emitted from a light emitting element (illumination A1), receiving light transmitted through the solution with a light receiving element (light reception A2), measuring the absorbance with monochromatic light selectively from the received transmitted light (measurement A3), and calculating a pH value corresponding to the measured absorbance based on a predefined absorbance table (calculation A4).

The pH measurement method according to the present embodiment will now be described sequentially.

In the present embodiment, the illumination A1 includes illuminating a solution containing a pH indicator and a pH changer with light emitted from the light emitting element. The illumination A1 is performed with a light emitting element such as a light-emitting diode (LED), but the structure is not limited.

The pH indicator herein refers to a reagent that changes its color in accordance with a pH value. Examples of the pH indicator include methyl orange, bromophenol blue, bromocresol green, bromothymol blue, phenol red, thymol blue, and phenolphthalein. The pH indicator may be added to a solution in advance to allow observation of a color change in accordance with a change in the pH value of the solution, or may be added to a solution immediately before the color of the solution is observed.

The pH changer herein refers to a substance that changes the pH value of a solution. Examples of the pH changer include a living substance such as a cell or living tissue. The pH changer may be a substance that either immediately or gradually change the pH value of a solution when added to the solution. The pH changer and the pH indicator may be added to a solution at the same time or at different times. For example, the pH changer may be added to a solution containing the pH indicator. The pH changer changes the pH value of the solution, and the pH indicator then changes its color accordingly. The pH changer may be cells, such as animal cells, plant cells, yeast cells, or bacterial cells. Examples of the animal cells include muscle cells, visceral cells such as the liver, blood cells such as lymphocytes, monocytes, and granulocytes, nerve cells, immune cells, and induced pluripotent stem (iPS) cells. These cells may be tissue-derived primary cells or may be subcultured cells. iPS cells have pluripotency and the self-renewal ability. iPS cells are produced by introducing several types of genes into somatic cells, such as skin cells of a human, and culturing the cells to be pluripotent cells that can differentiate into cells of various tissues and organs, similarly to embryonic stem (ES) cells. The self-renewal ability allows iPS cells to retain pluripotency after division and proliferation, thus achieving substantially unlimited proliferation. The pH measurement method and the pH measurement device according to the embodiments can be used for a solution containing iPS cells with high pluripotency, or more specifically a solution containing components with large changes.

The solution herein refers to a homogeneous liquid mixture and may be, for example, an aqueous solution, a methanol solution, and an ethanol solution. More specifically, the solution may be a buffer solution and a culture solution. The solution may contain an auxiliary substance in addition to the pH indicator and the pH changer. The auxiliary substance may be a metal ion such as phosphorus and sodium, a peptide, an amino acid, or a protein. The solution may be a culture solution. In this case, the culture solution provides a growth environment for a target in culturing biological tissues such as microorganisms and cells. The solution is a source of nutrients such as a carbon source including glucose, a nitrogen source including peptone and ammonium sulfate, and inorganic salts including amino acids, vitamins, and phosphate. The culture solution may be a liquid containing the above nutrients for cell culture or may be such a liquid additionally containing agar or gelatin.

The light reception A2 in the present embodiment includes receiving light transmitted through the solution with the light receiving element. The light reception A2 may be performed with an amorphous silicon positive-intrinsic-negative (PIN) photodiode as the light receiving element, but the structure is not limited.

The PIN photodiode is a three-layered photodiode with an intrinsic layer (I-layer) between a PN junction. The PIN photodiode includes three semiconductors, or p-type, intrinsic, and n-type (PIN) semiconductors, that are joined together. Whereas a PN photodiode includes a depletion layer with no electrons and no holes around a PN junction, a PIN diode includes a prepared I-layer with no electrons and no holes instead of a depletion layer.

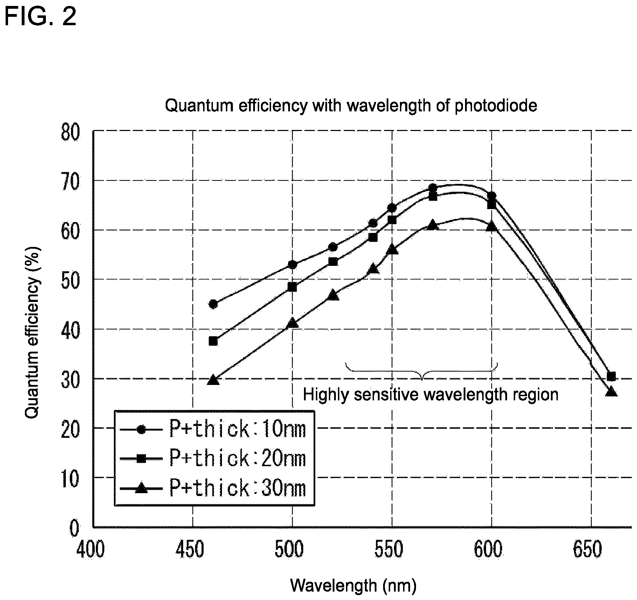

The materials for a photodiode may be silicon, germanium, indium, gallium, arsenic, and lead sulfide. These materials are selected as appropriate for the wavelength of light to be detected. Silicon absorbs light with wavelengths of 190 to 1,100 nm. Germanium absorbs light with wavelengths of 400 to 1,700 nm. Indium, gallium, and arsenic absorb light with wavelengths of 800 to 2,600 nm. Lead sulfide absorbs light with wavelengths of 1,000 to less than 3,500 nm. Amorphous silicon refers to non-crystalline silicon with a higher absorption coefficient than crystalline silicon. Amorphous silicon photodiodes have the highest quantum efficiency (internal quantum efficiency) at a wavelength of around 560 nm as shown in FIG. 2. In FIG. 2, the solid circles represent the quantum efficiency of a P+ layer with a thickness of 10 nm on a light receiving surface, solid squares represent the quantum efficiency of a P+ layer with a thickness of 20 nm, and solid triangles represent the quantum efficiency of a P+ layer with a thickness of 30 nm. As the thickness of the P+ layer increases, the quantum efficiency to light in wavelength regions excluding the wavelength region of green light may tend to decrease. Thus, the thickness of the P+ layer may be 10 nm or greater, specifically 20 nm or greater, and more specifically 20 to 30 nm.

The measurement A3 includes measuring the absorbance with monochromatic light selectively from the received transmitted light.

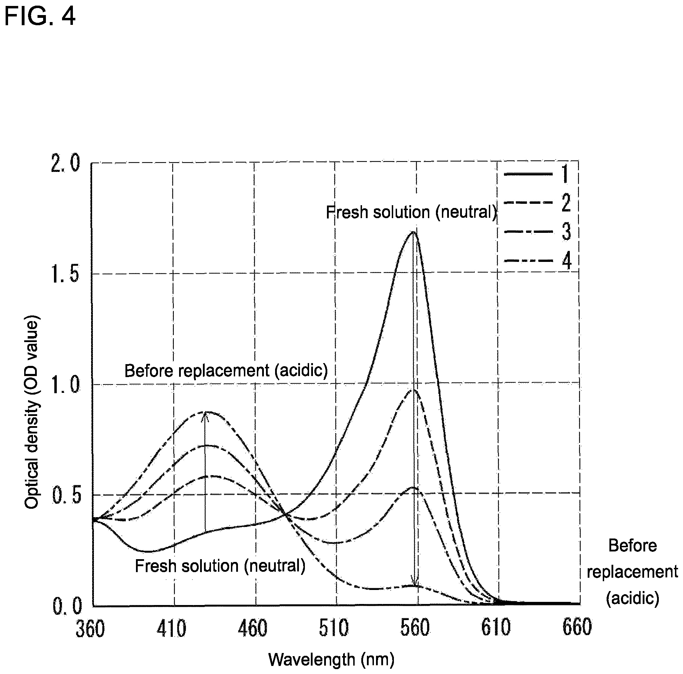

Monochromatic light herein refers to light with a single wavelength or in a predetermined wavelength region alone and not dividable further in the spectrum. The monochromatic light may be, for example, red light or light in a red wavelength region, blue light or light in a blue wavelength region, and green light or light in a green wavelength region. Monochromatic light may specifically be green light or light in a green wavelength region. The absorbance with green light or light in a green wavelength region may change largely in response to a change in the pH of a solution as shown in FIG. 4. The predetermined wavelength region is, for example, a wavelength region centered on a center wavelength. Light in the wavelength region is expressed using the center wavelength plus or minus the bandwidth of the wavelength. With the pH measurement method and the pH measurement device according to the embodiments, green light may have a wavelength of about 560 nm. A green wavelength region may have a wavelength region of about 560 nm ±40 nm, or about 560 nm ±20 nm. Receiving light in the wavelength region of monochromatic light improves the light sensitivity (the intensity of a signal received and converted photoelectrically).

In the present embodiment, the pH indicator may have a lower absorption peak in a predetermined wavelength region as the pH value of a solution decreases, and the light receiving element may have the highest quantum efficiency in the above predetermined wavelength region. This allows highly sensitive detection of the decrease in the absorption peak, thus allowing highly sensitive and accurate measurement of the pH value of a solution.

The calculation A4 includes calculating a pH value corresponding to the absorbance measured in the measurement based on a predefined absorbance table.

The absorbance table herein refers to data indicating the relationship between the pH value of each solution and the absorbance of each solution. The data is, for example, stored in a storage (memory). The storage may be a memory such as a storage circuit (memory circuit), an integrated circuit (IC), and a large-scale integration (LSI) included in a personal computer (PC), a stand-alone mass storage device, or a portable memory device such as a universal serial bus (USB) memory. The memory circuit and the memory may be a random-access memory (RAM) or a read-only memory (ROM).

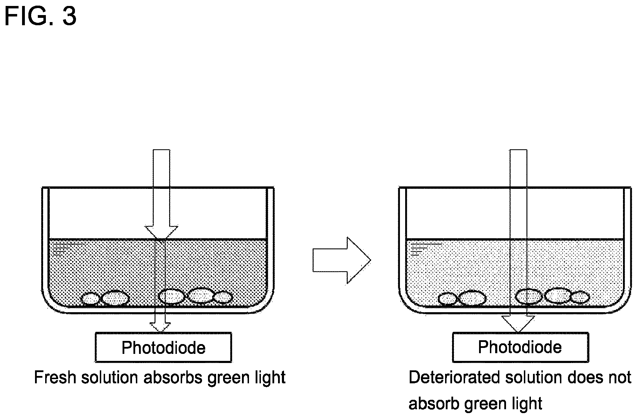

FIG. 3 is a schematic diagram of cell culture solutions used as the solution, phenol red used as the pH indicator, cells used as the pH changer, and an amorphous silicon photodiode used as the light receiving element in one embodiment of the present disclosure. Phenol red is a common pH indicator contained in a cell culture solution and has an absorption peak at wavelengths of around 430 to 440 nm and around 560 nm (FIG. 4). More specifically, for example, a 0.04 w/v% phenol red solution (FUJIFILM Wako Pure Chemical Corporation, with the molecular formula of $C_{19}H_{14}O_5S$ and the molecular weight of 354.38) may be used. The phenol red solution may be prepared by, for example, adding 14.2 ml of a 0.02 mol/L sodium hydroxide solution and water to 0.10 g of phenol red to obtain 250 ml of a phenol red solution. The cell culture solution containing phenol red is colored red in the optimal pH range of 6.8 to 7.2 (neutral) for a cell. However, as the pH decreases during cell culture, the solution has a higher absorption peak at a wavelength of around 430 to 440 nm and a lower absorption peak at a wavelength of around 560 nm. The solution thus changes to yellow. In FIG. 4, the absorbance of a fresh solution is indicated by 1 (solid line), the absorbance of the cell culture solution in which cells are cultured is indicated by 2 (dotted line) and 3 (dot-dash line), and the wavelength of the cell culture solution before replacement is indicated by 4 (two-dot chain line).

In the absorption spectrum of phenol red, the absorption with the green wavelength of 560 nm changes largely when the solution turns acidic. The PIN amorphous silicon photodiode has the highest quantum efficiency at a wavelength of around 560 nm. A color change can thus be detected without a color filter or a complicated structure. Light detected by the amorphous silicon photodiode is accumulated as electric charge. The amount of electric charge is then read to detect a color change.

The absorbance with green light is measured and compared with the predefined absorbance table. Table 1 below is an example of the absorbance table.

Table 1

| Light source | λ560 nm | | |
|---|---|---|---|
| pH | C1 | C2 | C3 |
| 7 | 1.80 | 1.70 | 1.6 |
| 6 | 1.60 | 1.55 | 1.48 |
| 5 | 1.51 | 1.46 | 1.41 |
| 4 | 1.42 | 1.38 | 1.33 |

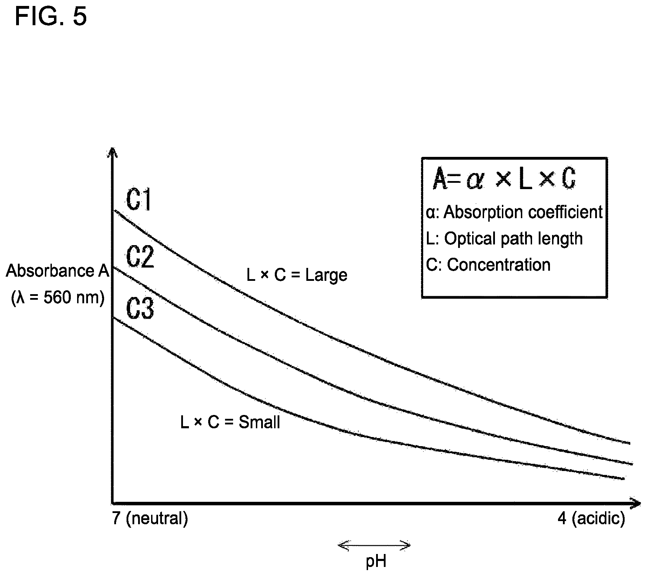

FIG. 5 shows the relationship between the pH of the cell culture solution and the absorbance at 560 nm. The absorbance A has the relationship represented by the Formula 1 below using the concentration C of phenol red.

$$A = \alpha \times L \times C \qquad (1)$$

In this formula, $\alpha$ is the absorption coefficient (mol absorption coefficient in l/mol·cm), and L is the optical path length in the solution. The absorbance A increases as the L × C increases. The absorbance A decreases as the L × C decreases. The absorption coefficient $\alpha$ includes the absorption coefficient of $\alpha HX$ of an acidic component (HX) of phenol red and the absorption coefficient $\alpha X$ of a base component (X⁻). The absorbance A at a wavelength $\lambda$ is the sum of the absorbance of the acidic component (HX) and the absorbance of the base component (X⁻). The sum is written by $A = \alpha HX \times [HX] + \alpha X \times [X^-]$, where [HX] is the concentration of the acidic component, and [X⁻] is the concentration of the base component. Although the concentration of phenol red C = [HX] + [X⁻] is constant, the concentration ratio between the acidic component (HX) and the base component (X⁻) changes as the pH changes, and the absorbance A also changes. In Table 1, L is about 0.1 to 10 mm and may be, for example, L = 3 nm. Further, C1, C2, and C3 have the relationship of C1 > C2 > C3. The concentration of phenol red in the phenol red solution may be set within the range of 0.01 to 0.1 w/v%. For example, C1 = 0.08 (w/v%), C2 = 0.05, and C3 = 0.02.

The initial absorbance of the cell culture solution and a change in the absorbance of the cell culture solution are measured based on the amount of change in absorbance caused by pH changes for the varying initial states of the several cell culture solutions (variations in L × C). This allows estimation of the pH value in each cell culture solution.

Modifications of the present embodiment may further include maintaining the pH changer in the solution before the illumination. This allows the pH changer to take time to sufficiently change the pH value of the solution.

Maintaining herein refers to maintaining the pH changer as it is in the solution or leaving the pH changer in the solution over time to sufficiently change the pH value of the solution.

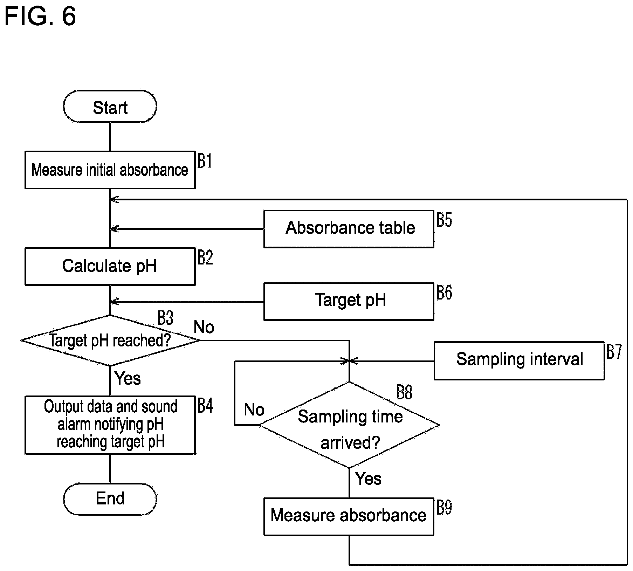

An example pH measurement method according to the first embodiment will now be described with reference to the flowchart shown in FIG. 6. In FIG. 6, Yes indicates an affirmative result (flag 1) in any determination, and No indicates a negative result (flag 0) in the determination.

The pH measurement method will now be described with reference to the flowchart.

In step B1, the initial absorbance of the solution is measured.

In step B2, the pH value corresponding to the measured absorbance is calculated based on the absorbance table in step B5.

In step B3, when the pH value corresponding to the measured absorbance reaches the target pH value read in step B6, the processing advances to step B4 and the data is output to sound an alarm notifying that the pH value has reached the target pH value. In some embodiments, an alarm may appear on an image display device (display) included in a PC either with or without an alarm sound.

An alarm may be transmitted to a mobile device such as a smartphone or a smartwatch carried by an operator of cell culture. The alarm may be transmitted to the mobile device by vibration, illumination, or a message pop-up.

When the pH value corresponding to the measured absorbance has not reached the target pH in step B3, the sampling interval is read based on a data table showing sampling intervals in step B7. In step B8, the determination is performed as to whether the sampling time has arrived, or in other words, the cell culture solution is to be replaced. The data table showing sampling intervals may be, for example, a time storage (time memory) storing information about sampling execution times or an elapsed-time storage (elapsed-time memory) storing information about elapsed times from the previous sampling execution time.

When the sampling time has arrived in step B8, the absorbance is measured in step B9. The processing then advances to step B2, where steps B3, B7, and B8 above are repeated until the pH reaches the target pH in step B3.

The pH measurement method according to the present embodiment with the above structure can effectively measure the pH without excess burden or time for the operator. Measurement Device

Second Embodiment

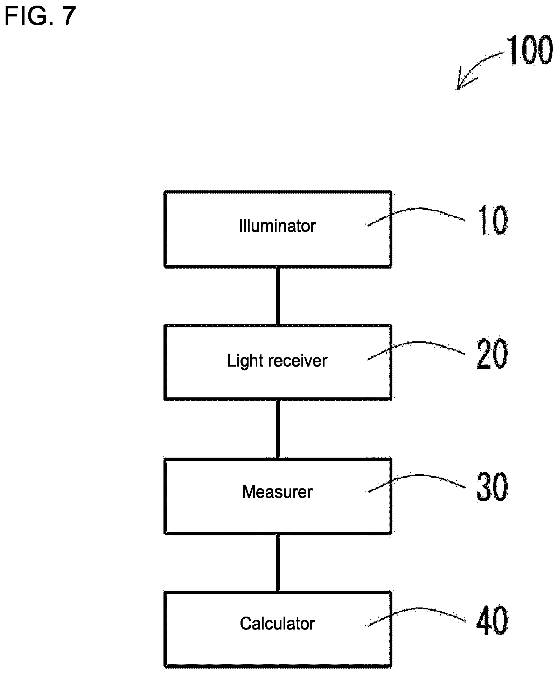

FIG. 7 is a functional block diagram of a pH measurement device 100 according to a second embodiment of the present disclosure. The measurement method according to the first embodiment of the present disclosure is performed by the pH measurement device 100.

The pH measurement device 100 according to the present embodiment includes an illuminator 10 that illuminates a solution containing a pH indicator and a pH changer with light emitted from a light emitting element, a light receiver 20 that receives light transmitted through the solution, a measurer 30 that measures the absorbance with monochromatic light selectively from the received transmitted light, and a calculator 40 that calculates a pH value corresponding to the measured absorbance measured by the measurer 30 based on a predefined absorbance table.

The illuminator 10 includes a light emitting element for illuminating a solution containing a pH indicator and a pH changer, but the structure is not limited. For example, the illuminator 10 may emit light in a wavelength region including monochromatic light and illuminate an object with the light. The illuminator 10 may be an LED illumination device, an electroluminescent device, a semiconductor laser device, or a fluorescent lamp.

The solution containing the pH indicator and the pH changer may be contained in a container formed from, for example, optically transparent glass or plastic. The container may be, for example, a Petri dish, a flask, or a microwell plate. The container for culturing cells may have any shape or size, but may have one or more spaces suitable for cell proliferation. For example, the container may be a Petri dish with a width or a diameter of about several centimeters to several tens of centimeters and a height of about several millimeters to several tens of centimeters, a flask with a width or a diameter of about several centimeters to several tens of centimeters and a height of about five to several tens of centimeters, or a microwell plate with a width or a diameter of about several centimeters to several tens of centimeters and a height of about 0.5 to several centimeters. The container is formed from, for example, optically transparent material, such as a plastic material or a glass material to be observable from outside. The microwell plate has wells each in the shape of, for example, a circle, a rectangle such as a square, or a polygon such as a pentagon or a hexagon in a plan view. The circular well is suitable for isometric proliferation of cells, allowing effective proliferation of the cells. The hexagonal well is suitable for the closest arrangement of wells, effectively reducing the size of the microwell plate.

The light receiver 20 may include, for example, a light receiving element that receives transmitted light, but the structure is not limited. The light receiver 20 includes a light receiving element such as a PIN photodiode with the structure described above. When the illuminator 10 is located above a container containing the solution including the pH indicator and the pH changer, the light receiver 20 may be located below the container. The light receiver 20 may include a light receiving element formed with a thin film formation method such as chemical vapor deposition (CVD) on a detection substrate, such as a glass substrate or a plastic substrate. The detection substrate may be placed inside the container on the bottom of the container.

In this case, the light receiving element, electrodes, and circuit wiring are placed on the detection substrate in the form of thin film. This allows the thinner and smaller light receiver 20 to be placed in the solution. A change in the pH value caused by the pH changer can be detected directly with high accuracy. The thinner and smaller light receiver 20 can be manufactured at low costs. The used light receiver 20 may be discarded when cell culture in the container has progressed sufficiently to end. An unused light receiver 20 may then be placed in the container for fresh cell culture using the container. In other words, the light receiver 20 may be replaced for every cell culture.

The detection substrate may be covered entirely with an insulating inorganic protective film formed from silicon nitride ($Si_3N_4$) or silicon oxide ($SiO_2$). This reduces contamination of the solution by the detection substrate. In this case, the used light receiver 20 may be washed and reused for cell culture.

The measurer 30 may include a signal converter that obtains a signal value predetermined based on a signal output from the light receiving element, but the structure is not limited. The measurer 30 obtains a signal output from the light receiving element through wires or wirelessly. For example, the measurer 30 may be an electronic device such as a PC including an analog-digital (AD) converter and a signal storage (signal memory). The AD converter obtains a signal (photoelectric conversion signal) corresponding to the absorbance with monochromatic light selectively from the transmitted light received by the light receiver 20 and performs AD conversion on the voltage value or current value of the signal. The signal storage stores a digital signal resulting from AD conversion.

The calculator 40 may include a computer that can calculate the pH value corresponding to the absorbance measured by the measurer 30 based on the predefined absorbance table, but the structure is not limited. For example, the calculator 40 may be a central processing unit (CPU) including an IC or LSI storing a computation software program included in an electronic device, such as a PC.

Third Embodiment

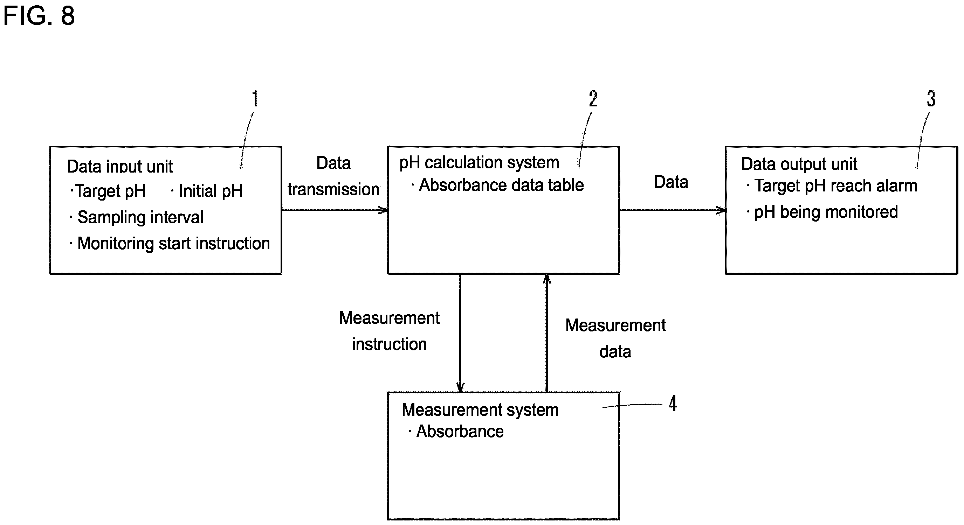

FIG. 8 is a block diagram of a pH measurement system 200 according to a third embodiment of the present disclosure. The measurement method according to the first embodiment of the present disclosure is performed by the pH measurement system 200.

The pH measurement system 200 includes a data input unit 1, a pH calculation system 2 connected to the data input unit 1, a data output unit 3 connected to the pH calculation system 2, and a measurement system 4 connected to the pH calculation system 2.

The data input unit 1 stores data about a target pH value, an initial pH value, a sampling interval, and an instruction to start monitoring. The data input unit 1 transmits the data to the pH calculation system 2.

The pH calculation system 2 stores the absorbance data table and transmits an instruction to start measurement to the measurement system 4. The pH calculation system 2 compares the absorbance measured by the measurement system 4 with values in the absorbance data table, and transmits the comparison data to the data output unit 3.

The data output unit 3 sounds an alarm when the comparison data indicates a value reaching a target pH value.

The measurement system 4 measures the absorbance of the solution upon receiving an instruction to start measurement from the pH calculation system 2. The measurement system 4 transmits the measured absorbance as measurement data to the pH calculation system 2.

Although the embodiments of the present disclosure have been described in detail, the present disclosure is not limited to the above embodiments, and may be modified or changed variously without departing from the spirit and scope of the present disclosure. The components described in the above embodiments may be entirely or partially combined as appropriate unless any contradiction arises.

Reference Signs List

1    data input unit
2    pH calculation system
3    data output unit
4    measurement system
10   illuminator
20   light receiver
30   measurer
40   calculator

**Claims**

1. A pH measurement method, comprising:

    illuminating a solution containing a pH indicator and a pH changer with light emitted from a light emitting element;
    receiving light transmitted through the solution with a light receiving element;
    measuring absorbance with monochromatic light selectively from the received transmitted light; and
    calculating a pH value corresponding to the measured absorbance based on a predefined absorbance table.

2. The pH measurement method according to claim 1, wherein

    the pH indicator has a lower absorption peak in a predetermined wavelength region in response to a decrease in the pH value of the solution, and the light receiving element has a highest quantum efficiency in the predetermined wavelength region.

3. The pH measurement method according to claim 2, wherein
    the pH indicator includes phenol red.

4. The pH measurement method according to claim 3, wherein
    the light receiving element includes an amorphous silicon photodiode with a positive-intrinsic-negative structure.

5. The pH measurement method according to any one of claims 2 to 4, wherein
    the monochromatic light includes light in the predetermined wavelength region.

6. The pH measurement method according to any one of claims 1 to 5, wherein
    the monochromatic light includes green light.

7. The pH measurement method according to claim 6, wherein
    the monochromatic light includes light in a wavelength region of 560 nm $\pm$40 nm.

8. The pH measurement method according to any one of claims 1 to 7, wherein
    the solution includes a culture solution.

9. The pH measurement method according to any one of claims 1 to 8, wherein
    the pH changer includes a living substance.

10. The pH measurement method according to claim 9, wherein
    the living substance includes a cell or tissue.

11. The pH measurement method according to claim 10, wherein
    the cell includes an induced pluripotent stem cell.

12. The pH measurement method according to any one of claims 1 to 11, further comprising:

maintaining the pH changer in the solution before the illuminating the solution.

13. A pH measurement device, comprising:

an illuminator configured to illuminate a solution containing a pH indicator and a pH changer with light emitted from a light emitting element; a light receiver configured to receive light transmitted through the solution; a measurer configured to measure absorbance with monochromatic light selectively from the received transmitted light; and a calculator configured to calculate a pH value corresponding to the absorbance measured by the measurer based on a predefined absorbance table.

14. The pH measurement device according to claim 13, wherein

the pH indicator has a lower absorption peak in a predetermined wavelength region in response to a decrease in the pH value of the solution, and the light receiving element has a highest quantum efficiency in the predetermined wavelength region.

15. The pH measurement device according to claim 14, wherein the pH indicator includes phenol red.

16. The pH measurement device according to claim 15, wherein the light receiving element includes an amorphous silicon photodiode with a positive-intrinsic-negative structure.

17. The pH measurement device according to any one of claims 13 to 16, wherein the monochromatic light includes light in a wavelength region of 560 nm $\pm$40 nm.

18. The pH measurement device according to any one of claims 13 to 17, wherein the pH changer includes an induced pluripotent stem cell.

19. The pH measurement device according to any one of claims 13 to 18, wherein

the light receiver includes a detection substrate and a light receiving element formed with a thin film forming method on the detection substrate, and the light receiver is located in the solution.

FIG. 1

```
        ┌──────────────┐
        │    Start     │
        └──────┬───────┘
               │
    ┌──────────┴──────────┐
    │    Illumination     │  A1
    └──────────┬──────────┘
               │
    ┌──────────┴──────────┐
    │   Light reception   │  A2
    └──────────┬──────────┘
               │
    ┌──────────┴──────────┐
    │    Measurement      │  A3
    └──────────┬──────────┘
               │
    ┌──────────┴──────────┐
    │    Obtainment       │  A4
    └──────────┬──────────┘
               │
        ┌──────┴───────┐
        │     End      │
        └──────────────┘
```

FIG. 2

Quantum efficiency with wavelength of photodiode

Legend:
- P+thick:10nm
- P+thick:20nm
- P+thick:30nm

Highly sensitive wavelength region

Y-axis: Quantum efficiency (%)
X-axis: Wavelength (nm)

# FIG. 3

Fresh solution absorbs green light

Deteriorated solution does not absorb green light

Photodiode

Photodiode

FIG. 4

FIG. 5

$$A= \alpha \times L \times C$$

α: Absorption coefficient
L: Optical path length
C: Concentration

Absorbance A
(λ = 560 nm)

C1

C2

C3

L × C = Large

L × C = Small

7 (neutral)

4 (acidic)

pH

# FIG. 6

```
                    ┌──────────────┐
                    │    Start     │
                    └──────┬───────┘
                           │
              ┌────────────────────────┐ B1
              │ Measure initial absorbance │
              └────────────┬───────────┘
                           │         ┌──────────────────┐ B5
                           │◄────────│ Absorbance table │
                           │         └──────────────────┘
              ┌────────────────────────┐ B2
              │      Calculate pH      │
              └────────────┬───────────┘
                           │         ┌──────────────────┐ B6
                           │◄────────│    Target pH     │
                           │         └──────────────────┘
                      B3       No
                   ╱ Target pH ╲────────────┐           ┌──────────────────┐ B7
                   ╲ reached?  ╱            │◄──────────│ Sampling interval │
                        │                   │           └──────────────────┘
                       Yes                  │    B8
              ┌────────────────────────┐ B4 │  No  ╱ Sampling time ╲
              │  Output data and sound │    └─────╲  arrived?     ╱
              │  alarm notifying pH    │           ╲             ╱
              │  reaching target pH    │                │
              └────────────┬───────────┘               Yes
                           │              ┌──────────────────┐ B9
                    ┌──────────────┐      │ Measure absorbance │
                    │     End      │      └──────────────────┘
                    └──────────────┘
```

FIG. 7

# FIG. 8

```
┌─────────────────────────────┐  1                ┌─────────────────────────────┐  2              ┌─────────────────────────────┐  3
│ Data input unit             │                   │ pH calculation system       │                 │ Data output unit            │
│ ·Target pH    · Initial pH  │   Data            │ · Absorbance data table     │   Data          │ · Target pH reach alarm     │
│ · Sampling interval         │   transmission    │                             │  ──────▶        │ · pH being monitored        │
│ · Monitoring start          │  ──────▶          │                             │                 │                             │
│   instruction               │                   │                             │                 │                             │
└─────────────────────────────┘                   └─────────────────────────────┘                 └─────────────────────────────┘
```

Measurement
instruction

Measurement
data

```
                                                  ┌─────────────────────────────┐  4
                                                  │ Measurement system          │
                                                  │ · Absorbance                │
                                                  │                             │
                                                  └─────────────────────────────┘
```

EP 4 039 790 A1

## INTERNATIONAL SEARCH REPORT

| International application No. |
| --- |
| PCT/JP2020/037239 |

### A. CLASSIFICATION OF SUBJECT MATTER

C12M 1/34(2006.01)i; G01N 21/17(2006.01)i; G01N 21/80(2006.01)i; G01N 33/48(2006.01)i; G01N 33/483(2006.01)i
FI: C12M1/34 A; G01N33/48 M; G01N33/483 C; G01N21/17 Z; G01N21/80
According to International Patent Classification (IPC) or to both national classification and IPC

### B. FIELDS SEARCHED

Minimum documentation searched (classification system followed by classification symbols)
C12M1/34; G01N21/17; G01N21/80; G01N33/48; G01N33/483

| Documentation searched other than minimum documentation to the extent that such documents are included in the fields searched | |
| --- | --- |
| Published examined utility model applications of Japan | 1922–1996 |
| Published unexamined utility model applications of Japan | 1971–2020 |
| Registered utility model specifications of Japan | 1996–2020 |
| Published registered utility model applications of Japan | 1994–2020 |

Electronic data base consulted during the international search (name of data base and, where practicable, search terms used)
JSTPlus/JMEDPlus/JST7580 (JDreamIII); CAplus/MEDLINE/EMBASE/BIOSIS/WPIDS (STN)

### C. DOCUMENTS CONSIDERED TO BE RELEVANT

| Category* | Citation of document, with indication, where appropriate, of the relevant passages | Relevant to claim No. |
| --- | --- | --- |
| X | JP 2012-170357 A (NIHON KOHDEN CORPORATION) 10 September 2012 (2012-09-10) claim 1, paragraphs [0001], [0007], [0034], [0042], [0043], [0045] | 1, 6-10, 13, 17 |
| Y | claim 1, paragraphs [0001], [0007], [0034], [0042], [0043], [0045] | 1-19 |
| Y | WO 2011/065057 A1 (SHARP CORP.) 03 June 2011 (2011-06-03) paragraph [0083], fig. 3 | 1-19 |
| Y | JP 2013-050364 A (FUJIFILM CORPORATION) 14 March 2013 (2013-03-14) paragraph [0040] | 1-19 |
| A | JP 64-035347 A (SUMITOMO ELECTRIC INDUSTRIES, LTD.) 06 February 1989 (1989-02-06) page 3, lower right column, lines 8-12 | 1-19 |

☐ Further documents are listed in the continuation of Box C.　　☒ See patent family annex.

| * | Special categories of cited documents: |
| --- | --- |
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
| --- | --- |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
| --- | --- |
| 25 November 2020 (25.11.2020) | 08 December 2020 (08.12.2020) |

| Name and mailing address of the ISA/ | Authorized officer |
| --- | --- |
| Japan Patent Office 3-4-3, Kasumigaseki, Chiyoda-ku, Tokyo 100-8915, Japan | |
| | Telephone No. |

Form PCT/ISA/210 (second sheet) (January 2015)

International application No.

PCT/JP2020/037239

| Patent Documents referred in the Report | Publication Date | Patent Family | Publication Date |
|---|---|---|---|
| JP 2012-170357 A | 10 Sep. 2012 | US 2012/0214250 A1 claim 1, paragraphs [0001], [0010], [0047], [0065], [0073], [0077] | |
| WO 2011/065057 A1 | 03 Jun. 2011 | US 2012/0241769 A1 paragraph [0110], fig. 3 | |
| JP 2013-050364 A | 14 Mar. 2013 | US 2013/0048864 A1 paragraph [0053] | |
| JP 64-035347 A | 06 Feb. 1989 | US 5003611 A column 3, lines 13-17 | |

Form PCT/ISA/210 (patent family annex) (January 2015)

**EP 4 039 790 A1**

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 5797911 B
- JP 2019106944 A
- JP 2019106945 A